# FASCICULE DE BREVET EUROPEEN

(11) **EP 2 583 662 B1**
(45) Date de publication et mention de la délivrance du brevet: **01.10.2014**
(21) Numéro de dépôt: 12187576.9
(22) Date de dépôt: 08.10.2012
(51) Int. Cl.: A61K 8/34, A61K 8/97, A61K 31/047, A61K 31/05, A61Q 19/00, A61K 36/16, A61P 17/10, A61K 9/00, A61K 47/10

(54) **Composition à base de meroterpene destinée aux peaux grasses, à tendance acneique ou atteintes d'acne**
Zubereitung enthaltend einen Meroterpen geeignet für fettige Haut mit einer Tendenz zur der Entwicklung von Akne
Composition comprising a meroterpen to manage oily skin with tendency to develop acne

(30) Priorité: 18.10.2011 FR 1159412
(43) Date de publication de la demande: 24.04.2013
(73) Titulaire: Thorel, Jean-Noël, 75014 Paris (FR)
(72) Inventeur: Thorel, Jean-Noël, 75014 Paris (FR)
(74) Mandataire: Buchet, Anne

(56) Documents cités:
- DE-A1- 3 417 234
- US-A1- 2006 099 284
- US-A1- 2006 251 749
- US-A1- 2011 117 036
- DATABASE TCM [Online] SIPO; 15 avril 2009 (2009-04-15), XP002675238, Database accession no. CN-101406513-A & CN 101 406 513 A (TIANJIN UNIVERSITY OF TRADITIO [CN] TIANJIN UNIVERSITY OF TRADITIONAL) 15 avril 2009 (2009-04-15)
- "A clinical study evaluating the dermatologic benefits of topical bakuchiol (UP256) cream on facial acne", JOURNAL OF THE AMERICAN ACADEMY OF DERMATOLOGY, C.V. MOSBY, ST. LOUIS, MO, US, vol. 64, no. 2, 1 février 2011 (2011-02-01), page AB19, XP027591310, ISSN: 0190-9622, DOI: 10.1016/J.JAAD.2010.09.099 [extrait le 2011-01-12]

## Description

### Domaine de l'invention

La présente invention a pour objet une composition à usage cosmétique ou dermatologique à base de méroterpène associé à un flavonoïde et un polyol.

Dans le cadre de la présente invention, il a été montré qu'une telle composition était particulièrement adaptée au traitement des peaux grasses ou à tendance acnéique, ou de l'acné à proprement parler.

De manière remarquable, une telle composition se caractérise par un panel d'actions complémentaires utiles dans ce type de traitement :
- effet protecteur du méroterpène sur l'oxydation du squalène ;
- action antimicrobienne, notamment vis à vis de la bactérie anaérobie gram positive *Propionibacterium acnes,* via un mécanisme direct et indirect (via des acides gras du sébum à activité antimicrobienne) ;
- effet anti-inflammatoire sur l'inflammation induite par *P. acnes* ;
- augmentation de la quantité de squalène non oxydé dans le sébum ;
- augmentation de la quantité d'acides gras du sébum impliqués dans la fonction barrière, contribuant notamment à limiter la pénétration des microorganismes et la comédogénicité ;
- diminution de la quantité d'acides gras du sébum impliqués dans la comédogénicité et l'irritation.

### Etat de la technique

L'acné est une pathologie inflammatoire chronique affectant le follicule pilo-sébacée, touchant le visage, le thorax et le dos. C'est une maladie extrêmement fréquente, puisqu'elle atteint près de 80% des adolescents.

Il existe différentes formes cliniques de l'acné liées aux populations concernées : l'acné polymorphe juvénile ou acné vulgaire qui peut être rétentionnel et/ou inflammatoire de sévérité et de durée variables ; l'acné de l'adulte retrouvé chez l'homme ou la femme classiquement de type inflammatoire ; l'acné du nouveau-né, du nourrisson et de l'enfant ; les formes sévères (acné nodulaire, acné conglobata, acné fulminans).

D'un point de vue clinique, l'acné est par définition une affection polymorphe qui associe des lésions rétentionnelles (microkystes, comédons), et/ou des lésions inflammatoires (papules, pustules ou nodules) qui peuvent coexister ou se succéder les unes aux autres lors de poussées. Sa sévérité varie des formes mineures à sévères.

La physiopathologie de l'acné implique 3 étapes qui évoluent dans le temps :
1) l'hypersécrétion sébacée, qui est le symptôme commun à toutes les formes d'acné ;
2) la formation de lésions rétentionnelles (la comédogenèse) : le canal excréteur du folicule pilo-sébacé en raison d'anomalies s'obstrue et l'hyperproduction de sébum par la glande sébacée ne peut plus être évacuée à la surface de l'épiderme, conduisant à une dilatation du follicule pilo-sébacé puis à la formation de lésions rétentionnelles (microcomédons infraclinique, évoluant vers un comédon macroscopique). Les comédons sont de 2 types :
   - les comédons ouverts (ou points noirs)
   - les comédons fermés (ou microkystes) : petite élevure blanchâtre de 2 à 3 mm de diamètre.
3) la formation de lésions inflammatoires où *P acnes,* qui prolifère dans les lésions rétentionnelles, joue un rôle majeur en produisant des substances inflammatoires, notamment IL-8 (Nagy *et al*., 2005) et TNF-α (Graham *et al*., 2004). Ceci entraine un afflux de polynucléaires neutrophiles responsables de la destruction de la paroi du follicule et une diffusion de l'inflammation dans les couches sous-jacentes. Ces lésions inflammatoires peuvent être superficielles (papules et pustules) ou profondes (nodules).

A un moindre degré de sévérité, les peaux à tendance acnéique sont, elles, caractérisées par la présence de peu de comédons éparpillés et de rares papules de petite taille.

La peau grasse ou hypersébacée est le symptôme commun à toutes les formes d'acné, caractérisée par une production excessive de sébum conduisant à un toucher gras, un aspect brillant avec des pores cutanés dilatés, en particulier dans la zone médiofaciale.

Le sébum est un produit de sécrétion gras, riche en acides gras et notamment en squalène, un carbure aliphatique à 30 atomes de carbone, précurseur du cholestérol. Le sébum joue un rôle positif important notamment dans la protection de la peau, mais il a également été établi qu'il existe une corrélation entre le taux de sécrétion de sébum et la sévérité de l'acné.

Par ailleurs, des études ont montré que certains composants du sébum étaient susceptibles de jouer un rôle actif dans l'acné.

Ainsi, la quantité de squalène est augmentée chez les patients acnéiques, où il est retrouvé sous forme oxydée. Or, le squalène oxydé est à l'origine de l'épaississement du sébum, de l'hyperkératinisation au niveau des follicules pilo-sébacés, de l'hyperprolifération de *P. acnes* et de l'induction de la réaction inflammatoire locale responsable du développement des lésions d'acné (Saint Léger *et al*., 1986 ; Chiba *et al*., 1999 et 2000 ; Ottaviani *et al*., 2006).

Pour limiter les effets potentiellement néfastes du squalène péroxydé, la peau adopte une stratégie d'auto-défense en libérant de la vitamine E à la surface de la peau. Or et pour des raisons encore inconnues, le niveau de vitamine E est réduit dans le sébum des patients acnéiques, renforçant ainsi le rôle de la péroxydation du squalène dans le développement de l'acné (Thiele *et al*., 1999 ; Picardo *et al*., 2009 ; Ottaviani *et al.,* 2010).

Par ailleurs, le sébum comprend des acides gras dotés d'une activité antibactérienne, notamment vis-à-vis de *P. acnes,* en particulier l'acide sapiénique qui est l'acide gras le plus abondant dans le sébum (Drake *et al*., 2008) et l'acide laurique (Nakatsuji *et al*., 2009). De plus, il a récemment été démontré que l'acide laurique stimule très fortement la synthèse du peptide anti-microbien HBD-2 dans les surnageants de sébocytes. Ces données indiquent que l'acide laurique possède une activité antimicrobienne directe sur *P acnes* et indirecte *via* l'augmentation des défenses innées de la peau au niveau des sébocytes (Nakatsuji *et al.,* 2010).

L'acide linoléique, également présent dans le sébum, est un acide gras essentiel qui contribue à l'intégrité de la barrière cutanée. Or, il a été décrit dans la littérature comme déficient dans les lésions d'acné. Cette réduction est associée à la sévérité de l'acné, dans la formation de comédons, la stimulation de la lipogenèse et la perte de fonction barrière pouvant être responsable de l'augmentation de la perméabilité cutanée aux microorganismes (Horrobin, 1989 ; Ottaviani *et al*., 2010).

Un autre exemple concerne l'acide oléique qui est un constituant du film hydrolipidique. Dans la peau acnéique, la présence d'acide oléique est augmentée. Or, celui-ci est comédogène (Choi *et al*., 1997 ; Katsuta *et al*., 2009) et irritant (Boelsma *et al.,* 1996).

Une première approche pour traiter l'acné consiste à contrôler la prolifération bactérienne notamment de *P. acnes.* Ainsi, des actifs comprenant du zinc sont préconisés pour leur activité bactéricide directe, souvent en combinaison avec un traitement antibiotique orale.

Par ailleurs, le document WO 2005/004891 décrit une association, pour application topique, d'agents antioxydants permettant de fluidifier le sébum et ainsi de diminuer les symptômes de l'acné.

Il existe actuellement une grande offre de traitements possibles qui présentent des limites, notamment concernant leur efficacité ou les effets secondaires associés. Il existe donc un besoin évident de développer de nouvelles solutions pour le traitement des peaux grasses voire de l'acné.

### Description de l'invention

Selon un premier aspect, la présente invention concerne une composition cosmétique ou dermatologique comprenant au moins un méroterpène associé à un flavonoïde et à un polyol.

Ainsi, la présente invention vise la combinaison de ces 3 actifs, à savoir un méroterpène, un flavonoïde et un polyol.

Un premier composant d'une telle composition est le méroterpène. Les méroterpènes sont des terpènes possédant un cycle aromatique et présentant la structure chimique suivante : 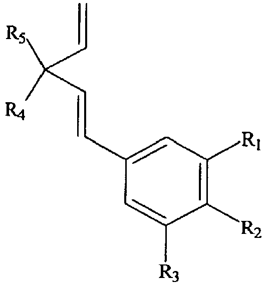 avec
R₁, R₂ et R₃ chacun indépendamment choisi dans le groupe constitué de H, OH, OR₆ ou CH₂R₆ avec R₆ est un alkyl branché ou linéaire en C₁-C₈ ;
R₄ et R₅ étant chacun indépendamment un groupe alkyl ou alkényl linéaire ou branché en C₁-C₂₀.

Des exemples privilégiés de méroterpènes sont le bakuchiol, composé dans lequel R₁=R₃=H, R₂=OH, R₄=CH₃ et R₅=CH₂CH₂CH=C(CH₃)₂, et la corylifoline, composé dans lequel R₁=R₃=H, R₂=OH, R₄=R₅=CH₃, ou des dérivés de ceux-ci.

Un méroterpène privilégié est le bakuchiol, par exemple le produit Sytenol^{®} A vendu par la société SYTHEON LTD. Dans ce produit, l'actif présente une pureté supérieure à 95%.

Les méroterpènes sont généralement obtenus à partir de plantes ou d'extraits de plante, même s'ils peuvent également être obtenus à partir de champignons ou produits synthétiquement. Des plantes ou extraits de plante servant de source pour les méroterpènes sont par exemple *Psoralea coryfolia*, *Psoralea grandulosa* et *Otholobium pubescens* (Fabaceae). En pratique, le méroterpène peut se présenter sous forme de matériel isolé ou purifié, de pureté au moins égale à 60% en poids, voire pur à au moins 95% en poids, ou peut être ajouté sous la forme d'un extrait de plante purifié comprenant avantageusement de 1 à 70% en poids de méroterpène par rapport au poids total de l'extrait.

Le méroterpène mis en oeuvre dans le cadre de l'invention est le bakuchiol. Avantageusement, le bakuchiol est isolé à partir de graines de *Psoralea coryfolia.*

De manière avantageuse, le méroterpène représente de 0,01 à 5% en poids de la composition, avantageusement de 0,1 à 2%, encore plus avantageusement 0,25%.

Comme il sera montré dans la suite de l'invention, la combinaison de cet actif avec les deux autres composants de la composition potentialise l'effet antibactérien notamment vis-à-vis de *P. acnes* du méroterpène. En pratique, ceci permet de diminuer sa concentration par rapport à son utilisation comme seul agent antimicrobien.

Le deuxième composant est un flavonoïde, largement distribué dans le règne végétal, notamment chez les plantes. Dans le cadre de la présente invention, la source de flavonoïdes est un extrait de ginkgo biloba. Il s'agit avantageusement d'un extrait de feuilles. Ainsi, le produit commercialisé par Greentech qui correspond à un extrait glycolique de feuilles de ginkgo biloba à 5% en matière sèche peut être utilisé dans le cadre de l'invention.

Avantageusement, la composition comprend un extrait végétal contenant le flavonoïde, ledit extrait étant présent à hauteur de 0,0001 à 20% en poids de la composition, avantageusement de 0,001 à 10% voire 2%, encore plus avantageusement 0,1%. On entend ici par «extrait végétal » la matière sèche comprenant le flavonoïde dans son solvant.

Une composition contient en outre un polyol, avantageusement du mannitol. Un autre polyol d'intérêt est le sorbitol, voire le xylitol.

Le mannitol mis en oeuvre peut par exemple être du mannitol en poudre, d'origine végétale (maïs, pomme de terre, blé), présentant une pureté supérieure à 98%, tel que commercialisé par IMCD sous la dénomination Pearlitol^{®}.

Dans le cadre de l'invention, le polyol, préférentiellement le mannitol, représente typiquement de 0,0001 à 10% en poids de la composition, avantageusement de 0,001 à 2%, encore plus avantageusement 0,01 %.

De manière privilégiée, la composition selon l'invention se présente sous une forme adaptée à l'administration par voie topique cutanée : crème, émulsion, H/E ou E/H, solution, suspension, gel, lait, lotion, en particulier crème, gel ou lotion.

Par conséquent, la présente composition peut contenir tout additif ou excipient adapté à sa formulation et à son application, tel que par exemple agents de mise en suspension, émulsifiants, polymères anioniques, cationiques ou non-ioniques, amphotères, protéines, vitamines tensioactifs, huiles minérales ou végétales, cires, gommes, résines, agents épaississants, acidifiants ou alcalinisants, solvants, stabilisateurs de pH, agents anti-UV, conservateurs, parfums, adjuvants classiques de la cosmétique et de la dermatologie.

La composition peut également contenir, outre les 3 ingrédients mentionnés ci-dessus, d'autres actifs tels qu'un agent antibactérien, notamment le zinc.

Des exemples de formulations cosmétiques sont les suivants :

### Formule 1

- Eau 41.23%
- Gélifiant 0.1 %
- Polymères 4.9%
- Emulsionnant 3%
- Glycols 12%
- Gluconate de zinc 3%
- AHA (acides de fruit) 20.5%
- Actifs divers 2.66%
- Bakuchiol 0.25%
- Extrait de Ginkgo biloba 0.1 %
- Mannitol 0.01%
- Corps gras 12%
- Parfum 0.25%

### Formule 2

- Eau 53.31%
- Régulateur de pH 3.43%
- AHA 17%
- Gluconate de zinc 3%
- Gélifiant 0.4%
- Silicones 6%
- Emulsionnant 3.5%
- Polymères divers 4%
- Actifs divers 0.92%
- Glycols 8%
- Bakuchiol 0.25%
- Extrait de Ginkgo biloba 0.1 %
- Mannitol 0.01%
- Parfum 0.08%

La présente invention a mis en évidence un certain nombre d'activités associées à une telle composition qui la rendent utilisable en tant que :
- agent fluidifiant du sébum, notamment grâce à son effet sur l'augmentation de la quantité de squalène non oxydé dans le sébum ;
- agent antimicrobien, notamment antibactérien vis-à-vis de *Propionibacterium acnes*, grâce à son effet bactéricide direct et à son effet indirect sur la stimulation ou la préservation de la quantité des acides gras du sébum à activité antimicrobienne (en particulier acides sapiénique et laurique) ;
- agent anti-inflammatoire vis-à-vis de l'inflammation induite par des bactéries, notamment *Propionibacterium acnes*, grâce à son effet inhibiteur sur la production de cytokines inflammatoires (en particulier IL-8 et de TNFα) ;
- agent protecteur de la fonction barrière de la peau, grâce à son effet stimulateur sur les acides gras du sébum impliqués dans l'intégrité de la fonction barrière tels que l'acide linoléique ;
- agent inhibiteur de la formation des comédons (anti-comédogène), notamment grâce à son effet inhibiteur sur les acides gras comédogènes du sébum tels que l'acide oléique ;
- agent contre l'irritation due à l'acné, notamment grâce à son effet inhibiteur sur les acides gras irritants du sébum tels que l'acide oléique.

L'ensemble de ces propriétés rend la composition selon l'invention adaptée au traitement des pathologies cutanées associées à la présence de *Propionibacterium acnes*, et plus généralement au traitement des peaux grasses ou à tendance acnéique, et de l'acné.

Un autre aspect de l'invention concerne un procédé de traitement cosmétique des peaux grasses ou à tendance acnéique, et de l'acné consistant à appliquer sur la peau ou le cuir chevelu une composition comme décrite précédemment.

### Description détaillée de l'invention

La manière dont l'invention peut être réalisée et les avantages qui en découlent, ressortiront mieux des exemples de réalisation qui suivent, donnés à titre indicatif et non limitatif, à l'appui des figures annexées.
La figure 1 représente la mesure de l'indice de protection (en %) du squalène par le Sytenol^{®}A (Bakuchiol) versus la vitamine E, à concentration équimolaire.
La figure 2 représente la croissance (en %) de *Propionibacterium acnes* en présence de différentes dilutions (D1 à D9) dans différents milieux :
   - en présence de S = Sytenol^{®}A.
   - en présence de Z=Zinc;
   - en présence de S + M + G = Sytenol^{®}A + Mannitol + flavonoïdes issus d'un extrait de Ginkgo biloba ;
   - en présence de S + M + G + Z = Sytenol^{®}A + Mannitol + flavonoïdes issus d'un extrait de Ginkgo biloba + Zinc
   - en présence d'un contrôle, à savoir du DMSO ayant servi de solvant pour la solubilisation du Sytenol^{®}A dans l'eau.

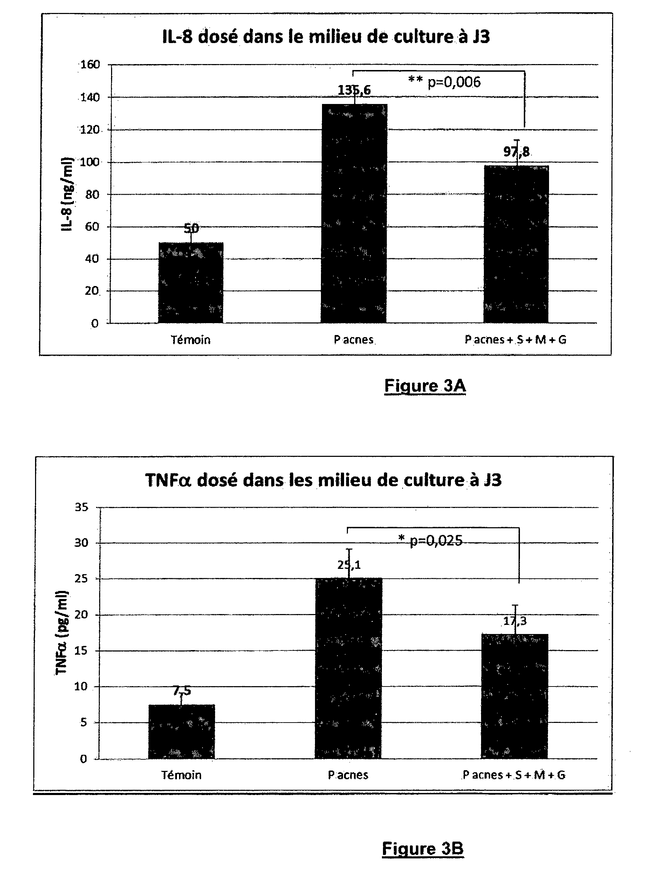

La figure 3 illustre A/ le dosage de IL-8 (ng/ml) et B/ de TNFα (ng/ml) dans les milieux de culture à J3, en l'absence ou en présence du noyau d'actifs selon l'invention (S+M+G).

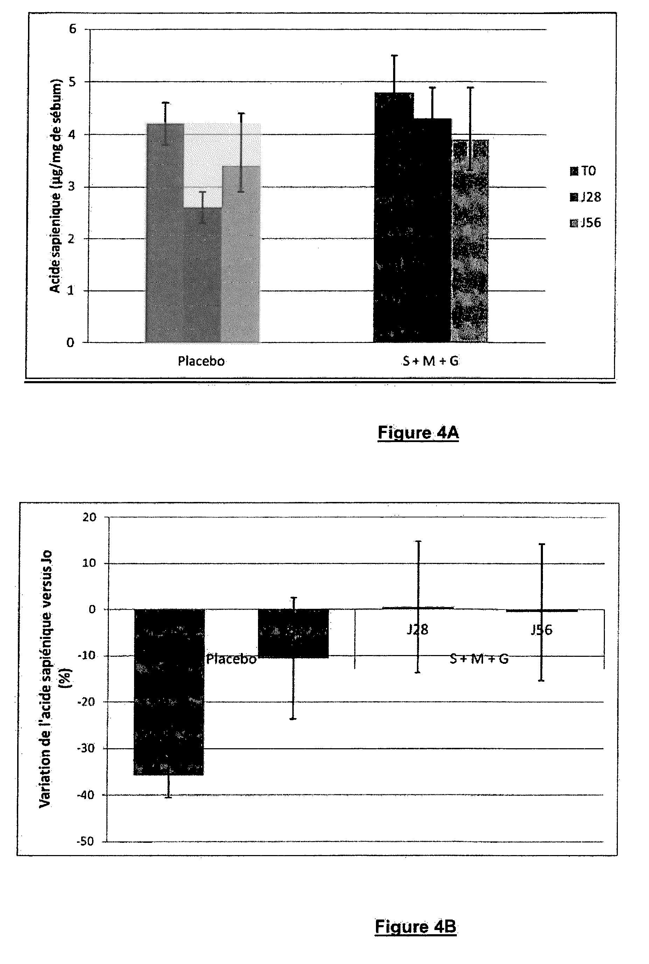

La figure 4 représente la mesure de l'acide sapiénique : A/ quantité d'acide sapiénique au cours du traitement par le noyau d'actifs selon l'invention (S+M+G) à T0, J28 et J56 *versus* placebo. B/ variation d'expression de l'acide sapiénique versus J0 après traitement par le noyau d'actifs selon l'invention (S+M+G) ou le placebo (résultats exprimés en pourcentage).

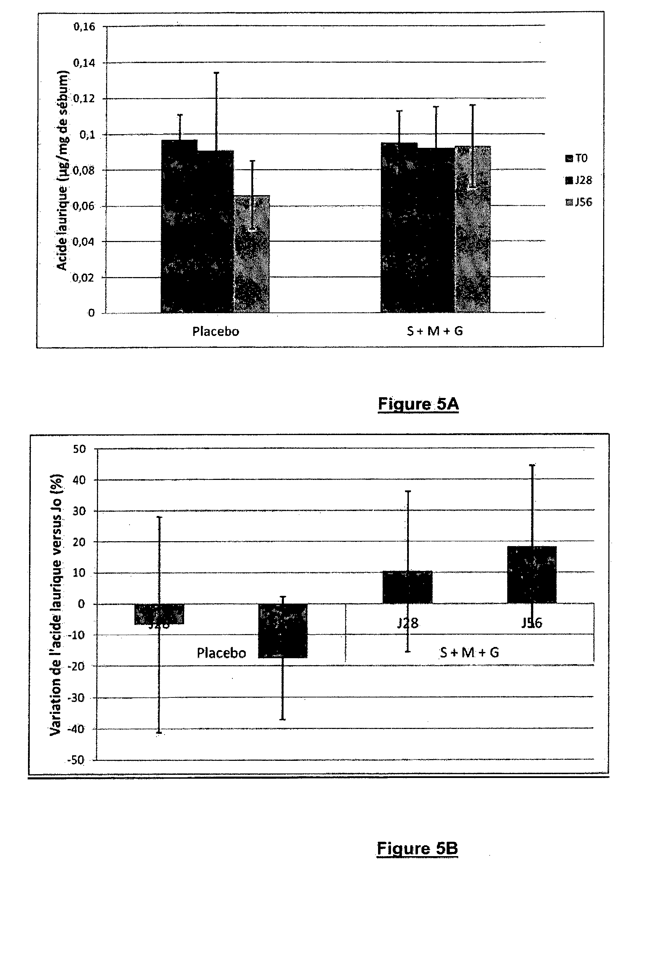

La figure 5 représente la mesure de l'acide laurique : A/ quantité d'acide laurique au cours du traitement par le noyau d'actifs selon l'invention (S+M+G) à T0, J28 et J56 *versus* placebo. B/ variation d'expression de l'acide laurique versus J0 après traitement par le noyau d'actifs selon l'invention (S+M+G) ou le placebo (résultats exprimés en pourcentage).

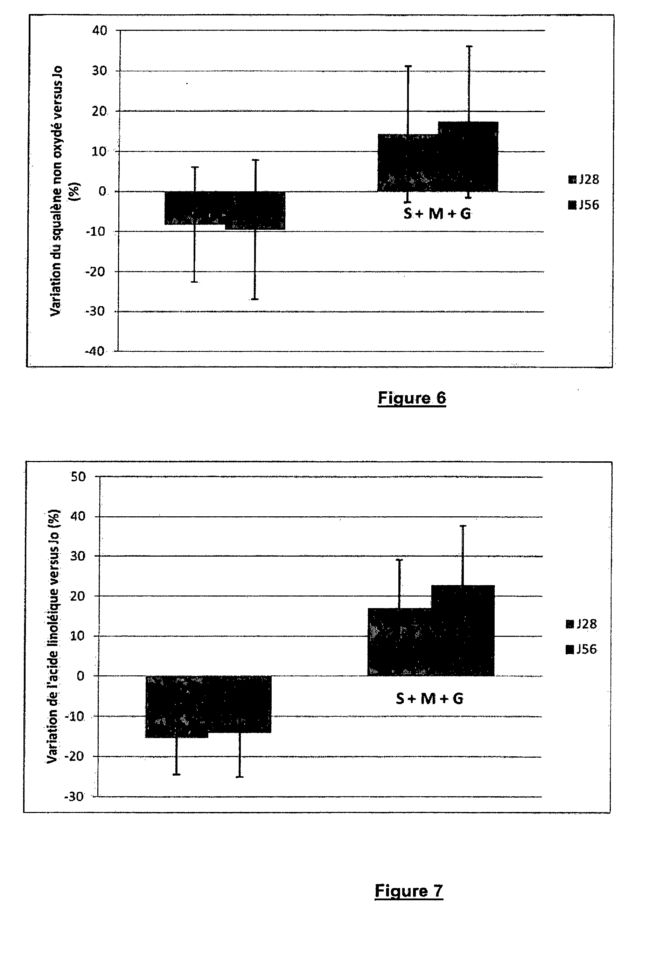

La figure 6 représente la mesure de la variation d'expression du squalène non oxydé versus J0, après traitement par le noyau d'actifs selon l'invention (S+M+G) ou le placebo (résultats exprimés en pourcentage).
La figure 7 représente la mesure de la variation d'expression de l'acide linoléique versus J0, après traitement par le noyau d'actifs selon l'invention (S+M+G) ou le placebo (résultats exprimés en pourcentage).

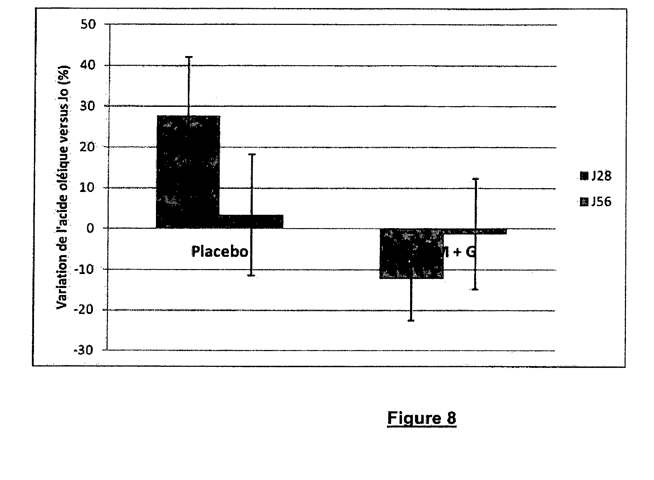

La figure 8 représente la mesure de la variation d'expression de l'acide oléique versus J0, après traitement par le noyau d'actifs selon l'invention (S+M+G) ou le placebo (résultats exprimés en pourcentage).

### A/ Nouvelle activité des méroterpènes : Effet protecteur vis-à-vis de l'oxydation du squalène

### 1/ But de l'étude :

Le but de l'étude est d'évaluer les effets d'un méroterpène, en l'occurrence le Sytenol^{®}A, sur l'oxydation du squalène induite par du peroxyde d'hydrogène (H₂O₂).

### 2/ Matériels et méthodes :

Le squalène (Sigma, St Quentin Fallavier) est mis en contact avec une quantité définie de peroxyde d'hydrogène pendant 1 heure pour induire son oxydation, en présence ou non du Sytenol^{®}A à 0.1%, 0.25% ou 0.5% (P/V). Un contrôle est réalisé avec un anti-oxydant lipophile de référence, la vitamine E, testée à 0,16% (3,8 mM), correspondant à une concentration équimolaire de Sytenol^{®}A à 0,1%.

A l'issue de l'oxydation, une étape d'extraction est réalisée, puis les échantillons sont analysés par chromatographie en phase gazeuse couplée à une analyse par spectrométrie de masse (GC-MS). Le squalène est dosé grâce à une gamme étalon (10 µg/ml à 2500 µg/ml). La quantité de squalène oxydée est déduite des dosages du squalène. Plus les quantités de squalène dosées sont importantes, moins il a été oxydé et inversement.

### 3/ Résultats :

Les résultats sont présentés dans le tableau ci-dessous :

| | **Indice de protection (%)** |
|---|---|
| **Echantillon oxydé** | **0.64** |
| **Echantillon non oxydé** | **100.00** |
| **Sytenol^{®}A à 0.1% (3.9mM)** | **30.02** |
| **Sytenol^{®}A à 0.25% (9.75 mM)** | **37.12** |
| **Vitamine E 0.16% (3.8mM)** | **15.17** |

Les résultats montrent que le Sytenol^{®}A permet de protéger l'oxydation du squalène induite par H₂O₂ et présente ainsi un pouvoir protecteur vis-à-vis de l'oxydation du squalène. Cet effet est dose-dépendant entre 0.1% et 0.25%.

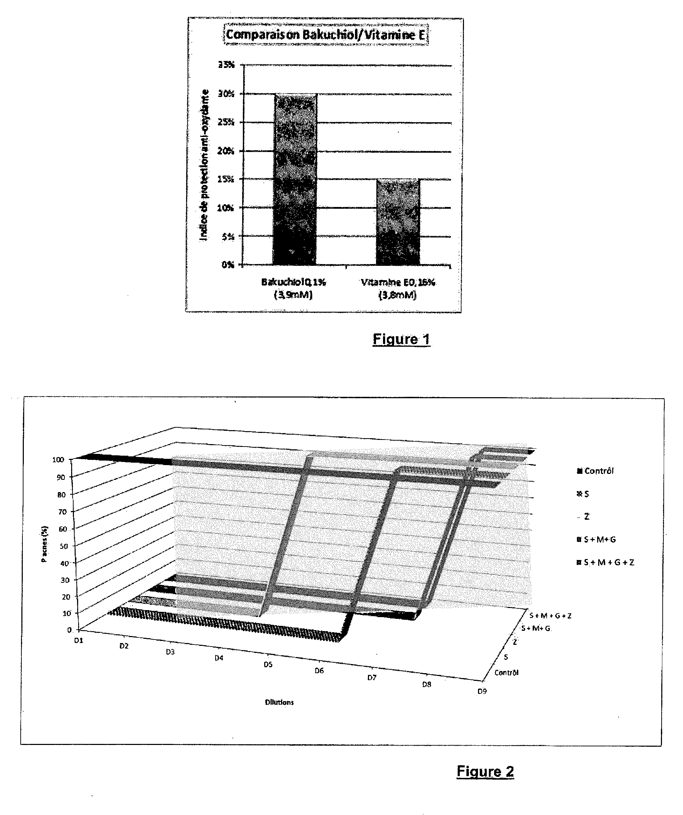

Comme illustré à la figure 1, à la concentration équimolaire de 3,8 mM, le Sytenol^{®}A est 2 fois plus efficace que la vitamine E en termes de protection de l'oxydation du squalène.

### B/ Activité du noyau d'actifs (S+M+G)

### I- ACTIVITE ANTIBACTERIENNE SUR Propionibacterium acnes

### 1/ But de l'étude :

L'objectif de cette étude est de déterminer la concentration minimale inhibitrice (CMI) d'actifs et de leur association en milieu solide, vis-à-vis de *Propionibacterium acnes.*

### 2/ Matériels et méthodes :

La détermination de la CMI des actifs à évaluer vis-à-vis de *Propionibacteriurn acnes* consiste, dans un premier temps, à préparer une gamme comportant neuf dilutions des actifs à tester. Chaque dilution est ensuite déposée en boîte de Pétri puis recouverte de gélose Wilkins-Chalgren. Après homogénéisation puis solidification, chacune des boîtes est inoculée avec une suspension calibrée (10⁷ UFC/mL) de *P acnes* (dépôt par strie), puis les boîtes sont incubées 18h à 37°C en anaérobiose. La souche utilisée est le *Propionibacterium acnes* CIP A179 (origine = glande sébacée (1946), source CRBIP, Institut Pasteur, Paris).

La préparation des géloses s'effectue à J1 en ajoutant 2 mL d'eau osmosée stérile dans une boîte de Pétri (témoin négatif) ou 2 ml de chaque dilution des gammes d'actifs 10 fois concentré et 18 mL de milieu gélosé. Après solidification à température ambiante, les boites sont séchées 30 min à 37°C puis ensemencées avec la suspension bactérienne calibrée (10⁷ UFC/mL) à l'aide d'une ose calibrée de 10 µL permettant de réaliser un dépôt correspondant à environ 10⁴ UFC).

La lecture des boites est effectuée après 18 heures de culture à 37°C permettant de déterminer la CMI correspondant à la plus faible concentration d'actif(s) qui inhibe toute culture visible de *P acnes.* La présence de 1 à 3 colonies n'est pas prise en considération.

Les produits évalués sont le S (Sytenol^{®}A, Sytheon), Z (Zinc gluconate, Azelis), seuls et les associations S + M (mannitol, IMCD/SPCI) + G (extrait glycolique de Ginkgo biloba, Greentech) et S + M + G + Z.

Le Sytenol^{®} A qui présente un caractère lipophile est dilué dans le DMSO (solution mère à 10%) avant d'être redilué dans l'eau osmosée. Le zinc, le mannitol et l'extrait de Ginkgo sont directement dilués dans l'eau osmosée.

Les essais ont également compris des contrôles en présence du solvant DMSO ayant servi à la solubilisation de S dans l'eau. L'érythromycine (2 à 0.007528 µg/ml) et le peroxyde de benzoyle (0.0128% à 0.00005%) ont été utilisés comme contrôles positifs.

Les gammes de dilutions testées sont :

| | **D1** | **D2** | **D3** | **D4** | **D5** | **D6** | **D7** | **D8** | **D9** |
|---|---|---|---|---|---|---|---|---|---|
| **S** | 0.25% | 0.1% | 0.01% | 0.002% | 0.001% | 0.0005% | 0.0001% | 0.00005% | 0.000025% |
| **M** | 0.01% | 0.004% | 0.0004% | 0.00008% | 0.00004% | 0.00002% | 0.000004% | 0.000002% | 0.000001% |
| **G** | 0.1% | 0.04% | 0.004% | 0.0008% | 0.0004% | 0.0002% | 0.00004% | 0.00002% | 0.00001% |
| **Z** | **3%** | **1.2%** | **0.12%** | **0.024%** | **0.012%** | **0.006%** | **0.0012%** | **0.0006%** | **0.0003%** |

### 3/ Résultats :

Les résultats des tests de croissance sont représentés dans la figure 2 et les valeurs de CMI dans le tableau ci-dessous :

| Produits testés | CMI |
|---|---|
| S | 0.001% |
| Z | 0.12% |
| S (+G à 0.0002% + M à 0.00002%) | 0.0005% |
| S (+G à 0.0002% + M à 0.00002% + Z à 0.006%) | 0.0005% |
| Peroxyde de Benzoyle | 0.008% |
| Erytromycine | 0.0000125% |

D'après les conditions expérimentales de cette étude, il apparaît que :
- la croissance n'est pas perturbée pas la présence du solvant DMSO aux concentrations présentes dans les dilutions du Sytenol ;
- Les produits Z et S seuls ont un pouvoir anti-bactérien vis-à-vis de *P acnes.*
- L'action anti-bactérienne de S sur *P acnes* est plus efficace que Z (CMI respective 0.001 % et 0.12%) ;
- L'association de G+M avec le produit S améliore l'efficacité anti-bactérienne de S ;
- L'association de Z au mélange (G+M+S) n'améliore pas l'efficacité anti-bactérienne vis-à-vis de P acnes de (S+G+M).

Il apparaît que l'effet bactéricide direct de la combinaison des 3 actifs (Sytenol^{®}A + Ginkgo biloba + mannitol) est meilleur que celui du Sytenol^{®} A seul, ce qui permet de diminuer la concentration en Sytenol^{®} A d'un facteur 2 environ dans la composition antibactérienne.

### II- EFFET ANTI-INFLAMMATOIRE SUR L'INFLAMMATION INDUITE PAR Propionibacterium acnes

### 1/ But de l'étude :

Le but de l'étude est d'évaluer, sur un modèle d'explants de peau humaine c'est-à-dire *in vivo,* l'activité anti-inflammatoire du noyau d'actifs (S + M + G).

### 2/ Matériels et méthodes :

Le noyau d'actifs est formulé dans une base neutre dans les proportions massiques suivantes :
- Sytenol^{®} A (S) : 0.25% ;
- extrait de Ginkgo biloba (Extrait Glycolique de feuilles, Greentech) (G) : 0.1%;
- mannitol (Pearlitol 060C, IMCD/SPCI) (M) : 0.01%.

La composition selon l'invention a la formule suivante:
- Eau 83.70974%
- Corps gras 10%
- Emulsionnant 3.5%
- Silicone 1%
- Epaississant 0.6%
- Gélifiant 0.1%
- Conservateurs 0.65%
- Régulateur de pH 0.00026%
- Parfum 0.08%
- Bakuchiol (S) 0.25%
- Extrait de Ginkgo biloba (G) 0.1%
- Mannitol (M) 0.01%

Le placebo a la formule suivante:
- Eau 84.06974%
- Corps gras 10%
- Emulsionnant 3.5%
- Silicone 1%
- Epaississant 0.6%
- Gélifiant 0.1%
- Conservateurs 0.65%
- Régulateur de pH 0.00026%
- Parfum 0.08%

Les explants de peau humaine, issus de déchets opératoires (11 mm de diamètre), sont mis en survie dans un milieu spécifique à 37°C.

Au jour 0 (J0), les explants sont traités par la formulation, à raison de 2 mg/cm² pendant 3 jours.

Aux jours J1 et J2, un disque de papier filtre imprégné de 30 µl de lyophilisat reconstitué de *P. acnes* est appliqué le matin, pendant 1 heure à la surface des explants, permettant de stimuler la synthèse de 2 cytokines inflammatoires : TNFα et IL-8. Des explants témoins, non traités par *P acnes* et traités par *P acnes* seulement, sont utilisés comme contrôles du test.

A J3, le milieu de culture de chaque explant est prélevé et conservé à -80°C. Les dosages de TNFα et d'IL-8 dans les surnageants sont réalisés par ELISA (ELISA TNFα, Cayman Chemical, et ELISA IL-8, RayBiotech). A l'issue du dosage, les DO à 412 nm et 450 nm sont lues, respectivement, et les concentrations des 2 cytokines sont calculées à partir des gammes étalons obtenues à partir des protéines recombinantes.

L'analyse statistique est réalisée grâce au test de Student dont le seuil de significativité est 0,05.

### 3/ Résultats :

Comme montré à la figure 3A, en présence du noyau d'actifs selon l'invention, la synthèse d'IL-8 est significativement réduite de 28% (**, p=0,006).

Comme montré à la figure 3B, en présence du noyau d'actifs selon l'invention, la synthèse de TNFα est significativement réduite de 31 % (*, p=0,026).

Ainsi, en réduisant à la fois la synthèse d'IL-8 et de TNFa, cytokines inflammatoires induites par *P acnes,* le nouveau noyau d'actifs selon l'invention réduit l'inflammation associée aux lésions d'acné.

### III- MODIFICATION DE LA COMPOSITION DU SEBUM

### 1 / But de l'étude :

Le but de l'étude clinique (*in vivo*) est d'évaluer les effets du nouveau noyau d'actifs selon l'invention sur la composition qualitative et quantitative des lipides du sébum en comparaison à un produit placebo.

### 2/ Matériels et méthodes :

Le placebo et la composition selon l'invention ont des formules comme indiquées ci-dessus au point II-2.

L'étude de l'effet du nouveau noyau d'actifs a été réalisée sur 2 groupes de 19 sujets à tendance acnéique. Un groupe a été traité avec le nouveau noyau d'actifs pendant 2 mois à raison de 2 applications par jour. Le groupe placebo a été traité dans les mêmes conditions avec l'excipient correspondant mais en l'absence du noyau d'actifs. Des prélèvements au niveau du front ont été réalisés à T0, J28 et J56 à l'aide d'une verrerie adaptée. Les lipides ont été récupérés à l'aide d'un solvant organique inerte et stockés à -20°C en attendant l'analyse biochimique.

La quantification des lipides du sébum a été réalisée par chromatographie en phase gazeuse couplée à une analyse par spectrométrie de masse (GC/MS). Différentes méthodes analytiques ont été réalisées dans le but de déterminer les acides gras totaux, le squalène, les stérols, les cires, les glycérides et les acides gras libres.

### 3/ Résultats :

### 1) Modification de l'expression d'acides gras à activité antimicrobienne :

Parmi les acides gras dont l'expression est modulée au cours du traitement par le noyau d'actifs, 2 sont connus comme possédant des propriétés antimicrobiennes puissantes : l'acide sapiénique et l'acide laurique.

### 1.1. L'acide sapiénique

On constate à la figure 4A que le taux d'acide sapiénique est augmenté de 37% par rapport au placebo à J28 et de 10% à J56. Ainsi, le traitement des volontaires avec le nouveau noyau d'actifs permet de préserver le taux d'acide sapiénique par rapport à J0, alors qu'il est réduit dans le groupe placebo (Figure 4B). Par conséquent, le noyau d'actifs selon l'invention exerce une action antibactérienne indirecte, en préservant le taux d'acide sapiénique dans le sébum cutané.

### 1.2. L'acide laurique

On constate à la figure 5A que le taux d'acide laurique est augmenté de 36% à J56. Ainsi, le traitement des volontaires avec le nouveau noyau d'actifs permet de stimuler le taux d'acide laurique par rapport à J0, alors qu'il est réduit dans le groupe placebo à J28 et J56 (Figure 5B). Par conséquent, le noyau d'actifs selon l'invention exerce une action antibactérienne indirecte, en stimulant le taux d'acide laurique dans les peaux à tendance acnéique.

L'ensemble de ces résultats montre qu'en plus des propriétés antimicrobiennes directes sur *P. acnes* (cf. point 1 ci-dessus), le nouveau noyau d'actifs selon l'invention exerce une activité antimicrobienne indirecte, en stimulant l'acide laurique et l'acide sapiénique qui possèdent tous les 2 des propriétés antimicrobiennes vis-à-vis de *P. acnes.* De plus, l'acide laurique est capable d'exercer une activité antimicrobienne indirecte en stimulant la synthèse de peptides antimicrobiens (HBD-2) dans les cultures de sébocytes. Ainsi, le noyau d'actifs selon l'invention possède une activité anti-*P acnes* multifactorielle.

### 2) Modification de l'expression du squalène :

On constate à la figure 6 qu'après 56 jours de traitement, le squalène non oxydé augmente de 17% par rapport à J0 et de 27% par rapport au placebo. Ainsi, en favorisant le squalène non oxydé, le noyau d'actifs selon l'invention contribue *in vivo* à améliorer les propriétés du sébum notamment sa fluidité.

### 3) Modification de l'expression de l'acide linoléique

On constate à la figure 7 qu'après 56 jours de traitement, l'acide linoléique qui est déficient, augmente de 22,8% par rapport à J0 et de 37% par rapport au placebo. En stimulant l'acide linoléique, le noyau d'actifs selon l'invention contribue *in vivo* à renforcer la fonction barrière de l'épiderme et à limiter la comédogénicité.

### 4) Modification de l'expression de l'acide oléique

On constate à la figure 8 qu'après 28 jours de traitement, l'acide oléique est réduit de 12% par rapport à J0 et de 40% par rapport au placebo. En réduisant l'acide oléique, le noyau d'actifs selon l'invention contribue *in vivo* à limiter la comédogénicité et l'irritation cutanée associée à l'acné.

Différents marqueurs tels que l'acide laurique, l'acide sapiénique, l'acide linoléique, l'acide oléique et le squalène non oxydé sont modulés favorablement après traitement des volontaires à peau grasses par le noyau d'actifs selon l'invention. Ces résultats obtenus *in vivo* montre que le noyau d'actifs selon l'invention modifie la qualité et la quantité du sébum conduisant à une réduction de la comédogénicité, une réduction de la lipogenèse, une augmentation de la fonction barrière et une stimulation des défenses anti-microbiennes permettant de réduire le développement de *P acnes.*

### REFERENCES

Boelsma E, Tanojo H, Boddé HE, Ponec M. Assessment of the potential irritancy of oleic acid on hunian skin: Evaluation in vitro and in vivo. Toxicol In Vitro. 1996 Dec;10(6):729-42
Chiba K et al. Comedogenicity of squalene monohydroperoxide in the skin after topical application. J Toxicol Sci 2000; 25: 77-83.
Chiba K, Sone T, Kawakami K, Onoue M. Skin roughness and wrinkle formation induced by repeated application of squalene-monohydroperoxide to the hairless mouse. Exp Dermatol. 1999 Dec;8(6):471-9.
Choi EH, Ahn SK, Lee SH. The changes of stratum corneum interstices and calcium distribution of follicular epithelium of experimentally induced comedones (EIC) by oleic acid. Exp Dermatol. 1997 Feb;6(1):29-35.
Drake DR, Brogden KA, Dawson DV, Wertz PW. Thematic review series: skin lipids. Antimicrobial lipids at the skin surface. J Lipid Res. 2008 Jan;49(1):4-11. Epub 2007 Sep 28. Review.
Graham GM, Farrar MD, Cruse-Sawyer JE, Holland KT, Ingham E. Proinflammatory cytokine production by human keratinocytes stimulated with Propionibacterium acnes and P. acnes GroEL. Br J Dermatol. 2004 Mar;150(3):421-8.
Horrobin DF. Essential fatty acids in clinical dermatology. J Am Acad Dermatol. 1989 Jun;20(6):1045-53. Review.
Katsuta Y, Iida T, Hasegawa K, Inomata S, Denda M. Function of oleic acid on epidermal barrier and calcium influx into keratinocytes is associated with N-methyl D-aspartate-type glutamate receptors. Br J Dermatol. 2009 Jan;160(1):69-74. Epub 2008 Sep 19.
Nagy I, Pivarcsi A, Koreck A, Széll M, Urbán E, Kemény L. Distinct strains of Propionibacterium acnes induce selective human beta-defensin-2 and interleukin-8 expression in human keratinocytes through toll-like receptors. J Invest Dermatol. 2005 May;124(5):931-8
Nakatsuji T, Kao MC, Fang JY, Zouboulis CC, Zhang L, Gallo RL, Huang CM. Antimicrobial property of lauric acid against Propionibacterium acnes: its therapeutic potential for inflammatory acne vulgaris. J Invest Dermatol. 2009 Oct;129(10):2480-8. Epub 2009 Apr 23.
Nakatsuji T, Kao MC, Zhang L, Zouboulis CC, Gallo RL, Huang CM. Sebum free fatty acids enhance the innate immune defense of human sebocytes by upregulating beta-defensin-2 expression. J Invest Dermatol. 2010 Apr;130(4):985-94. Epub 2009 Dec 24.
Ottaviani M, Alestas T, Flori E, Mastrofrancesco A, Zouboulis CC, Picardo M. Peroxidated squalene induces the production of inflammatory mediators in HaCaT keratinocytes: a possible role in acne vulgaris. J Invest Dermatol. 2006 Nov;126(11):2430-7. Epub 2006 Jun 15.
Ottaviani M, Camera E, Picardo M. Lipid médiators in acne. Mediators Inflamm. 2010;2010. pii: 858176. Epub 2010 Aug 25. Review.
Picardo M, Ottaviani M, Camera E, Mastrofrancesco A. Sebaceous gland lipids. Dermatoendocrinol. 2009 Mar;1(2):68-71.
Saint-Léger D et al. A possible role for squalene in the pathogenesis of acne. In vivo study of squalene oxides in skin surface and intra-comedonal lipids of acne patients. Br J Dermatol 1986; 114: 543-552.
Thiele JJ, Weber SU, Packer L. Sebaceous gland secretion is a major physiologie route of vitamin E delivery to skin. J Invest Dermatol. 1999 Dec;113(6):1006-10.

## Revendications

1. Composition à usage cosmétique et/ou dermatologique comprenant ;
- du bakuchiol ;
- un flavonoïde contenu dans un extrait de ginkgo biloba ; et
- un polyol.

2. Composition selon la revendication 1 c*aractérisée* en ce que le bakuchiol représente de 0,01 à 5% en poids de la composition, avantageusement de 0,1 à 2%, encore plus avantageusement 0,25%.

3. Composition selon la revendication 1 ou 2 ***caractérisée* en ce que** l'extrait de ginkgo biloba contenant le flavanoïde représente de 0,0001 à 20% en poids de la composition, avantageusement de 0,001 à 2%, encore plus avantageusement 0,1%.

4. Composition selon l'une des revendications précédentes ***caractérisée* en ce que** le polyol est du mannitol.

5. Composition selon l'une des revendications précédentes ***caractérisée* en ce que** le polyol est du xylitol.

6. Composition selon l'une des revendications précédentes ***caractérisée* en ce que** le polyol représente de 0,0001 à 10% en poids de la composition, avantageusement de 0,001 à 2%, encore plus avantageusement 0,01%.

7. Composition selon l'une des revendications précédentes ***caractérisée* en ce qu'**elle se présente sous la forme d'une crème, d'un gel ou d'une lotion.

8. Utilisation cosmétique d'une composition selon l'une des revendications 1 à 7 comme :
- agent anti-oxydant du squalène ; et/ou
- agent fluidifiant du sébum ; et/ou
- agent protecteur de la fonction barrière de la peau ; et/ou
- agent inhibiteur de la formation des comédons.

9. Composition selon l'une des revendications 1 à 7 pour son utilisation comme :
- agent antimicrobien, notamment antibactérien vis-à-vis de *Propionibacterium acnes* ; et/ou
- agent anti-inflammatoire vis-à-vis de l'inflammation induite par des bactéries, notamment *Propionibacterium acnes* ; et/ou
- agent contre l'irritation liée à l'acné.

10. Procédé de traitement cosmétique des peaux grasses ou à tendance acnéique consistant à appliquer sur la peau ou sur le cuir chevelu une composition selon l'une des revendications 1 à 7.

11. Composition selon l'une des revendications 1 à 7 pour son utilisation dans le traitement de l'acné.

## Patentansprüche

1. Zusammensetzung zur kosmetischen und / oder dermatologischen Verwendung, umfassend;
- Bakuchiol;
- Eine in einem Extrakt aus Ginkgo biloba enthaltenen Flavonoide; und
- Ein Polyol.

2. Zusammensetzung nach Anspruch 1, **dadurch gekennzeichnet, dass** das Bakuchiol 0,01 bis 5 Gew.-%, vorzugsweise 0,1 bis 2 Gew.-%, bevorzugter 0,25 Gew.-% der Zusammensetzung ausmacht.

3. Zusammensetzung nach Anspruch 1 oder 2, **dadurch gekennzeichnet, dass** der Flavonoide enthaltende Ginkgo-biloba-Extrakt 0,0001 bis 20 Gew.-%, vorzugsweise 0,001 bis 2%, noch bevorzugter 0,1 % der Zusammensetzung ausmacht.

4. Zusammensetzung nach einem der vorhergehenden Ansprüche, **dadurch gekennzeichnet, dass** das Polyol Mannitol ist.

5. Zusammensetzung nach einem der vorhergehenden Ansprüche, **dadurch gekennzeichnet, dass** das Polyol Xylitol ist.

6. Zusammensetzung nach einem der vorhergehenden Ansprüche, **dadurch gekennzeichnet, dass** das Polyol 0,0001 bis 10% Gew.-%, vorzugsweise 0,001 bis 2 Gew.-%, noch bevorzugter 0,01 Gew.-% der Zusammensetzung ausmacht.

7. Zusammensetzung nach einem der vorhergehenden Ansprüche, **dadurch gekennzeichnet, dass** sie in Form einer Creme, eines Gels oder einer Lotion vorliegt.

8. Kosmetische Verwendung einer Zusammensetzung nach einem der Ansprüche 1 bis 7 als:
- Squalenantioxidans; und / oder
- Verdünnungsmittel von Talg; und / oder
- Schutzmittel der Barrierefunktion der Haut; und / oder
- Bildung von Komedonen hemmendes Mittel.

9. Zusammensetzung nach einem der Ansprüche 1 bis 7 zur Verwendung als:
- antimikrobielles Mittel, insbesondere antibakterielles Mittel gegen *Propionibacterium acnes;* und / oder
- entzündungshemmendes Mittel gegen die durch Bakterien, insbesondere durch *Propionibacterium acnes,* hervorgerufene Entzündung; und / oder
- Mittel gegen aknebedingte Reizung.

10. Verfahren zur kosmetischen Behandlung von fettiger oder Akne neigender Haut, das das Aufbringen auf die Haut oder auf die Kopfhaut eine Zusammensetzung nach einem der Ansprüche 1 bis 7 umfasst.

11. Zusammensetzung nach einem der Ansprüche 1 bis 7 zur Verwendung in der Behandlung von Akne.

## Claims

1. Composition for cosmetic and/or dermatological use comprising:
- bakuchiol;
- a flavonoid contained in a ginkgo biloba extract; and
- a polyol.

2. Composition according to claim 1 **characterized in that** the bakuchiol represents from 0.01 to 5% in weight of the composition, preferably from 0.1 to 2% and even more preferably 0.25%.

3. Composition according to claim 1 or 2 **characterized in that** the extract of ginkgo biloba that contains the flavonoid represents from 0.0001 to 20% in weight of the composition, preferably from 0.001 to 2%, and even more preferably 0.1 %.

4. Composition according to any of the preceding claims **characterized in that** the polyol is mannitol. 1.

5. Composition according to any of the preceding claims **characterized in that** the polyol is xylitol. 1.

6. Composition according to any of the preceding claims **characterized in that** the polyol represents from 0.0001 to 10% in weight of the composition, preferably from 0.001 to 2%, and even more preferably 0.01 %.

7. Composition according to any of the preceding claims **characterized in that** it is in the form of a cream, gel or lotion.

8. Cosmetic use of a composition according to any of claims 1 to 7 as:
- a squalene antioxidant agent; and / or
- a sebum thinning agent; and / or
- a protective agent for the barrier function of the skin; and / or
- an inhibitor agent of the formation of comedones.

9. Composition according to any of claims 1 to 7 for its use as:
- an antimicrobial agent, especially against *Propionibacterium acnes;* and / or
- an anti-inflammatory agent against inflammation induced by bacteria, in particular *Propionibacterium acnes;* and / or
- an agent against acne related irritation.

10. Method for the cosmetic treatment of greasy or acne-prone skins consisting of applying to the skin or to the scalp a composition according to any of claims 1 to 7.

11. Composition according to any of claims 1 to 7 for its use in the treatment of acne.
